## Europäisches Patentamt

## European Patent Office

## Office européen des brevets

(11) Veröffentlichungsnummer: **0 063 649** **B1**

(12) # EUROPÄISCHE PATENTSCHRIFT

(45) Veröffentlichungstag der Patentschrift:
04.09.85

(51) Int. Cl.⁴: **A 61 B 5/02**

(21) Anmeldenummer: **81110789.5**

(22) Anmeldetag: **28.12.81**

(54) Messeinrichtung zur nichtinvasiven Feststellung venöser bzw. arterieller Abfluss- und Durchflussstörungen.

(30) Priorität: **12.01.81 DE 3100610**

(43) Veröffentlichungstag der Anmeldung:
**03.11.82 Patentblatt 82/44**

(45) Bekanntmachung des Hinweises auf die Patenterteilung:
**04.09.85 Patentblatt 85/36**

(84) Benannte Vertragsstaaten:
**AT BE CH DE FR GB IT LI NL SE**

(56) Entgegenhaltungen:
**CH - A - 621 056**
**DE - A - 1 541 170**
**FR - A - 2 216 972**
**FR - A - 2 473 873**
**GB - A - 1 538 695**
**GB - A - 2 061 496**
**US - A - 3 769 974**
**US - A - 3 871 362**
**US - A - 3 973 251**

**Die Akte enthält technische Angaben, die nach dem Eingang der Anmeldung eingereicht wurden und die nicht in dieser Patentschrift enthalten sind.**

(73) Patentinhaber: **Blazek, Vladimir, Dr.-Ing., Butzweide 14, D-5100 Aachen (DE)**
Patentinhaber: **Wienert, Volker, Prof.Dr.med., Elsenborn 53, D-5100 Aachen (DE)**

(72) Erfinder: **Blazek, Vladimir, Dr.-Ing., Butzweide 14, D-5100 Aachen (DE)**
Erfinder: **Wienert, Volker, Prof.Dr.med., Elsenborn 53, D-5100 Aachen (DE)**

(74) Vertreter: **Hansmann, Dierk, Dipl.-Ing., Jessenstrasse 4, D-2000 Hamburg 50 (DE)**

ACTORUM AG

**Beschreibung**

Die Erfindung bezieht sich auf eine Messeinrichtung zur nichtinvasiven Feststellung peripherer Abfluss- und Durchflussstörungen in menschlichen Extremitäten, wobei die Blutentleerung bzw. -auffüllung der Venen oder Arterien eines Extremitätenareals in ihrem zeitlichen Verlauf erfassbar ist, mit einem leichten, an einem zu untersuchenden Hautareal mit doppelt klebenden Folienringen zu befestigenden Messkopf, der Öffnungen aufweist, in denen ein optischer Strahlungsempfänger und eine Strahlungsquelle angeordnet sind, wobei der Strahlungsempfänger und die Strahlungsquelle unmittelbar die Hautoberfläche berühren, und mit einer Auswerteschaltung für das Ausgangssignal des Strahlungsempfängers.

Eine gattungsgemässe Einrichtung ist aus der Druckschrift «Cardiopulse Analyser» der FRICAR AG, Zürich/Schweiz, bekannt. In der klinischen Praxis besteht das Problem, eine objektive Erfassung der Hautdurchblutungsänderung unter Belastung einerseits und eine Erkennung von venösen Strömungshindernissen (Thrombosen) im Bereich der Extremitäten andererseits durchzuführen.

Bei einer chronisch venösen Insuffizienz (CVI) liegt eine periphere venöse Abflussstörung vor, bedingt durch eine valvuläre Dysfunktion. Unter dem Begriff CVI werden die Folgezustände der primären wie auch sekundären Varikose subsummiert. Das klinische Bild der CVI ist vielgestaltig. Man findet Gefässveränderungen (Corona phlebectatica paraplantaris, Besenreiser, Capillaritis alba usw.), Ödeme, Hautveränderungen (z.B. Hyper- oder Depigmentierung) und Hautdefekte. In der klinischen Praxis hat sich folgende subjektive, rein deskriptive Einteilung der CVI bewährt.

Grad I: Corona phlebectatica paraplantaris,
Grad II:Hyper- oder Depigmentierungen mit oder ohne Corona phlebectatica,
Grad III:Florides oder abgeheiltes Unterschenkelgeschwür.

Weil diese Stadieneinteilung jedoch wenig Aussage über die wirkliche Dysfunktion der Venen zulässt, müssen Messverfahren zur Diagnostik zugezogen werden. In den letzten 10 Jahren wurden entscheidende Fortschritte auf dem Gebiet der angiologischen Messtechnik erzielt. Nach dem heutigen Stand der Technik können folgende messtechnische Methoden zur Beurteilung von peripheren Venenerkrankungen angewendet werden (siehe Ehringer et al.: Venöse Abflussstörungen. F. Enke Verlag, Stuttgart 1979):

1. Venendruckmessung (Phlebodynamometrie),
2. Volumenmessung (Plethysmographie),
3. Phlebographie (auch Isotopenphlebographie),
4. Radio-Jod-Fibrinogen-Test,
5. Thermographie.

Insbesondere die Messmethodik der invasiven, dynamischen Venendruckmessung findet in den letzten Jahren breite klinische Anwendung, da sie es erlaubt, den Grad der venösen Insuffizienz objektiv zu beurteilen.

Der Venendruck an beliebiger Stelle des menschlichen Körpers ist eine zusammengesetzte Grösse, die sich in Horizontallage bei stehender Flüssigkeit aus dem lokalen Flüssigkeitsdruck (hämostatischer Druck) und dem Dehnungswiderstand der Gefässwand (Gefässtonus) ergibt. Zu diesem Grunddruck kommt – bei einsetzender Strömung – der Strömungsdruck hinzu, dessen Höhe von der Herzleistung und peripherem Widerstand bestimmt ist.

Zu diesem, in horizontaler Ruhelage im ganzen Venensystem annähernd gleich hohen und fast konstanten Venendruck addiert sich schliesslich – im Stehen – der hydrostatische Druck. Er entspricht rechnerisch dem Abstand, der zwischen der Messebene liegt und der hydrostatischen Indifferenzebene, die etwa in Höhe des vierten Interkostalraums links anzunehmen ist.

Durch die CVI vergrössert sich das Volumen der Extremität bzw. der venöse Druck. Da eine gute Korrelation zwischen Volumen und Druck z.B. von THULESIUS 1973 nachgewiesen wurde (s. obige Literaturstelle, S. 462, Abb. 232) wird die dynamische Druckmessung in peripheren subkutanen Venen zur Beurteilung der CVI hinzugezogen.

Im allgemeinen wird die venöse Druckmessung folgendermassen durchgeführt:

Eine Fussrückenvene wird mit einer Injektions-Kanüle punktiert, an die ein Polyäthylenschlauch als Verbindungsstück zum elektronischen Druckwandler angeschlossen wird. Die Kanüle wird bei richtiger Lage auf der Haut fixiert. Auf einem Registriergerät (meist EKG-Schreiber) wird ein Ruhedruck $P_o$ registriert. Danach wird ein sog. Bewegungsprogramm (bewährt haben sich 10 Zehestände oder 10 Dorsalflexionen des Fusses in 15 sec) durchgeführt. Durch die Muskelkontraktion (Wadenmuskelpumpe, Sprunggelenkspumpe) wird infolge der Bewegung das Blut aus der Extremität vermehrt abgeführt; dies führt unterhalb des Kniegelenkes zu einer Druck- und Volumensenkung. Das Absinken des Ausgangsdruckes (Ruhedruck) innerhalb des Bewegungsprogramms wird registriert. Der tiefste Druck wird mit $P_{min}$ bezeichnet. Anschliessend wird das Bein in Ruhe gehalten. In einer Wiederauffüllphase (Zeitspanne $t_o$) steigt der Druck wieder von $P_{min}$ auf $P_o$.

Auf diesem Effekt basieren die Methoden der elektrischen Rheographie und der Impedanz-Plethysmographie. Bei diesen Verfahren werden auf die Haut mehrere metallische Kontakte aufgelegt.

Bei der sog. photoelektrischen Plethysmographie (vergl. z.B. Acta Dermatovener [Stockholm], 50 [1970], 263–269 oder Phys. Med. Biol. 19 [1974], 317–328) wird die Pulswelle (Änderung des peripheren Blutvolumens in Abhängigkeit von der Herzfunktion) ermittelt. Dabei werden überwiegend Finger- oder Zehenspitzen mit Hilfe einer Lichtquelle durchstrahlt; die transmittierten oder reflektierten, sich mit dem Herzrhythmus periodisch verändernden Lichtanteile werden erfasst. So kann z.B. die Zahl der Herzschläge pro Minute nichtinvasiv ermittelt werden.

Alle plethysmographischen Methoden weisen aber mehrere, in der einschlägigen Literatur beschriebene Nachteile auf und haben sich in der klinischen Praxis wegen störender Nebeneffekte, geringer Messgenauigkeit, schlechter Reproduzierbarkeit bzw. wegen der komplizierten Handhabung nicht durchsetzen können. Deshalb sind die plethysmographischen Methoden weniger verbreitet als die blutige Venendruckmessung. Auch die Ultraschall-Doppler-Technik ist ein nichtinvasives qualitatives Verfahren zur Beurteilung der venösen Hämodynamik. Hier wird die zeitabhängige Blutgeschwindigkeit registriert; quantitative Aussagen über CVI sind nicht möglich. Schliesslich haben auch die invasiven Methoden der Phlebographie, des Radio-Jod-Fibrinogen-Tests und der Thermographie wegen der grossen Körperbelastung, des apparativen Aufwandes und begrenzter Aussagekraft nur in Spezialkliniken eine Anwendung gefunden. Sie werden erfolgreich zur Diagnostik der Venenthrombose eingesetzt; eine quantitative Beurteilung der CVI ist aber nicht möglich.

Es ist allgemein bekannt, dass es sich bei der CVI um eine venöse Abflussstörung im Bereich der grossen Transportvenen, also um einen Makrozirkulationsschaden handelt. Nicht bekannt geworden ist hingegen, dass hierbei auch ein Mikrozirkulationsschaden im Kapillargebiet vorliegt. Alle Symptome der CVI sind deshalb im Bereich der Kutis und Subkutis lokalisiert.

Bei arteriellen Durchblutungsstörungen wird die Hautfarbe und die Venenfüllung durch Lagewechsel der entsprechenden Körperpartien in charakteristischer Weise (siehe Widmer: Arterielle Durchblutungsstörungen in der Praxis, H. Hübner Verlag 1972) verändert und nach bisheriger Praxis visuell durch den Arzt diagnostiziert. Diese Diagnose ist subjektiv. Sie kann auch durch äussere Untersuchungsbedingungen, z.B. durch die Beleuchtungsverhältnisse beeinflusst werden.

Aus der FR-A-2 216 972 ist auch eine Anordnung zur photoelektrischen Pulsumschreibung zu entnehmen, die auf die Erfassung der Blutvolumenänderung in der Mikrozirkulation (Kapillargefässe unmittelbar unter der Hautoberfläche) infolge der quasiperiodischen Änderung des arteriellen Blutdruckes basiert. Sie dient der Aufnahme der Pulswelle auf dem optischen Wege mit dem Ziel, entweder die Herztätigkeit durch Messung der Pulsfrequenz zu überwachen, oder durch Registrieren der Pulswelle die Aussagen über die arterielle Pulsform und die arterielle Pulswellenlaufzeit zu gewinnen. Diese Grössen werden auch oft zur Bestimmung der arteriellen Blutdrucke herangezogen.

Die Aufgabe der erfindungsgemässen Messeinrichtung besteht darin, eine quantiative Beurteilung peripherer venöser Abflussstörungen und arterieller Durchblutungsstörungen in menschlichen Extremitäten zu gewährleisten sowie auch die Diagnose der arteriellen Durchblutungsstörungen infolge langsamer Entleerung- bzw. Füllvorgänge der Blutgefässe der Haut zu ermöglichen.

Diese Aufgabe wird erfindungsgemäss durch die kennzeichnenden Merkmale des Patentanspruches 1 gelöst. Vorteilhafte Aus- und Weiterbildungen der Erfindung sind in den Unteransprüchen angegeben.

Die erfindungsgemässe Messeinrichtung zur nichtinvasiven Licht-Reflexions-Rheographie (LRR) beruht auf einer Erfassung und Auswertung der von mehreren selektiven Strahlungsquellen in die Haut eingestrahlten und von dem dermalen Gefässplexus zurück zu einem Photodetektor reflektierten Strahlungsanteile, gegebenenfalls unter der gleichzeitigen Messung der Hauttemperatur.

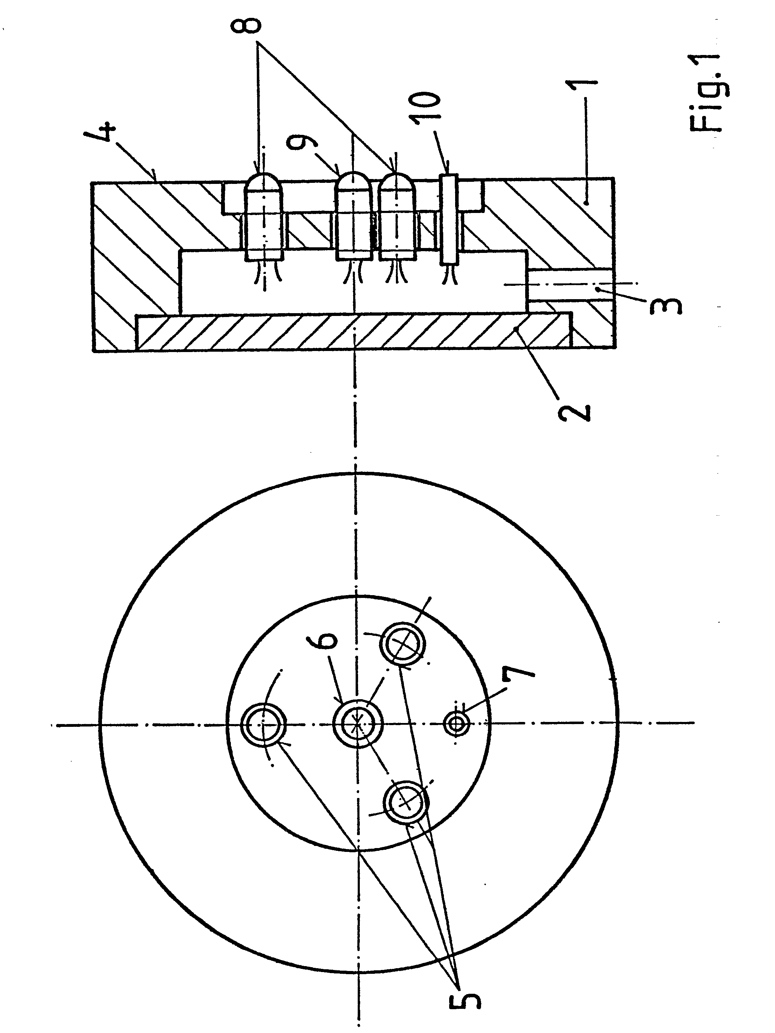

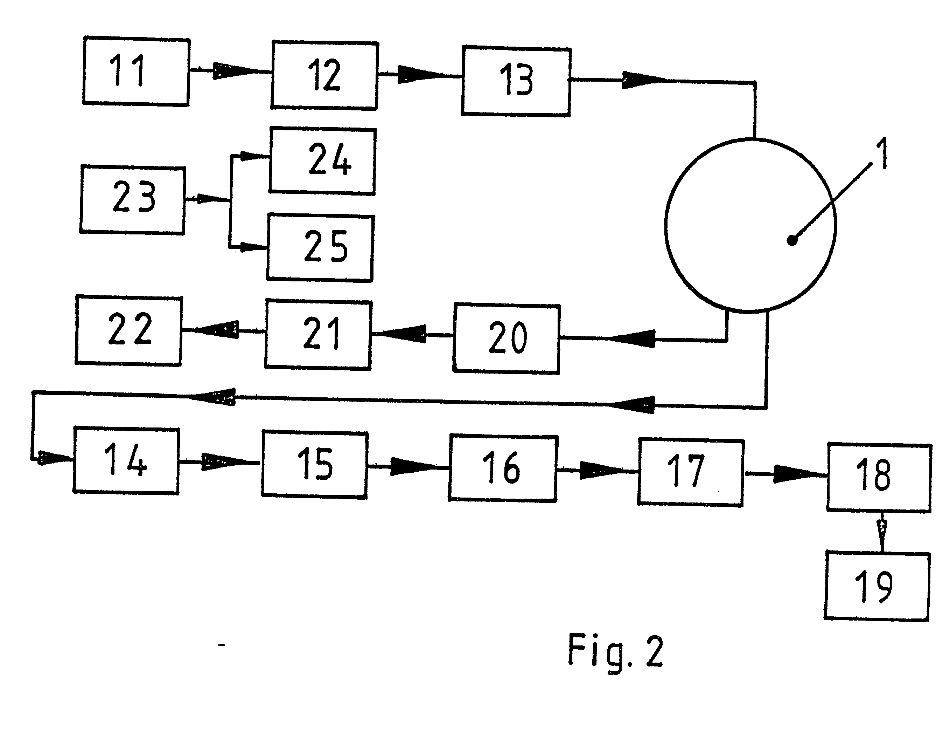

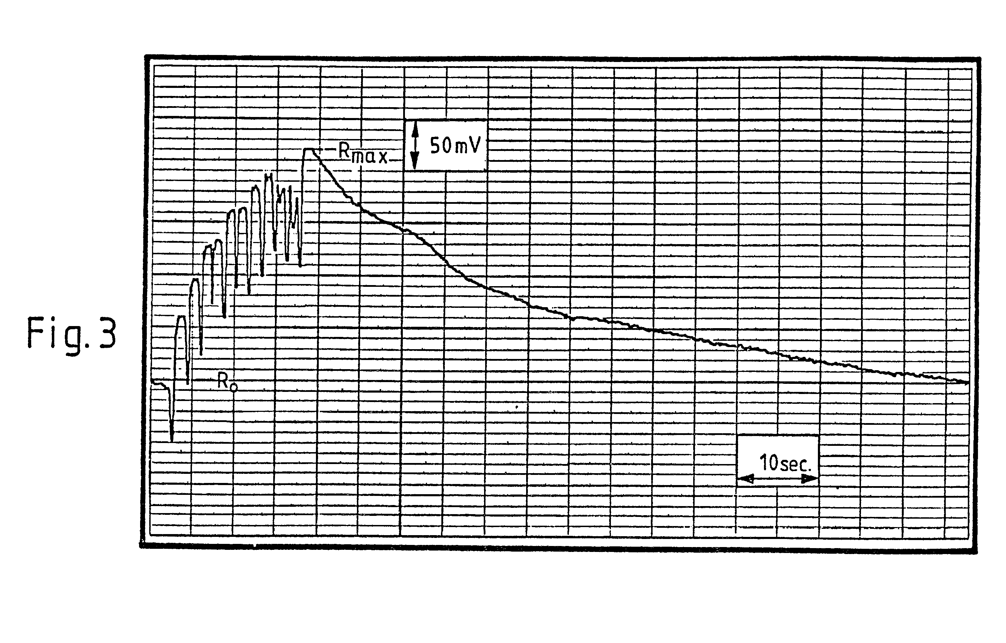

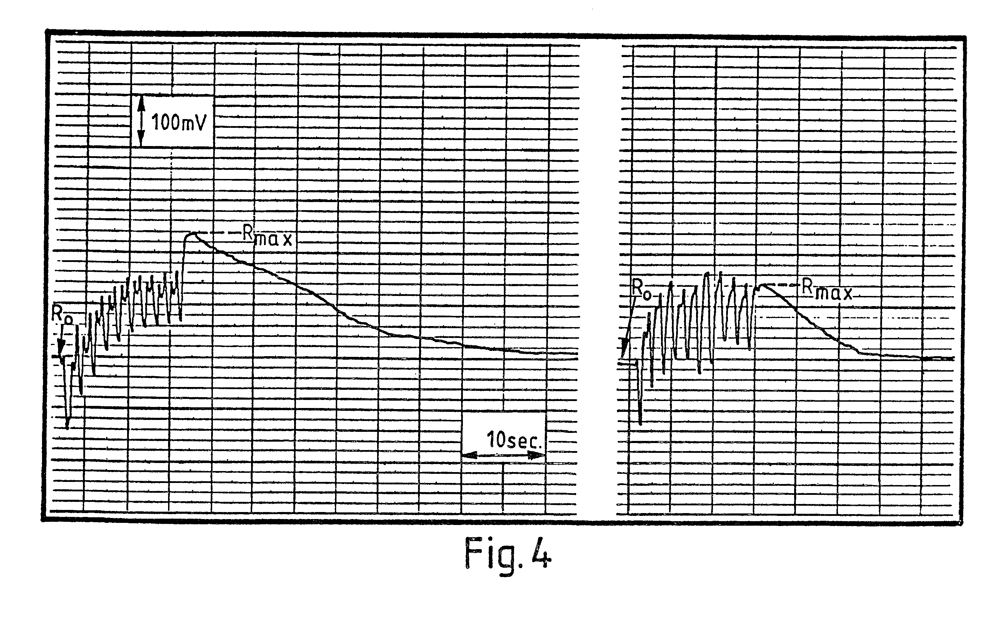

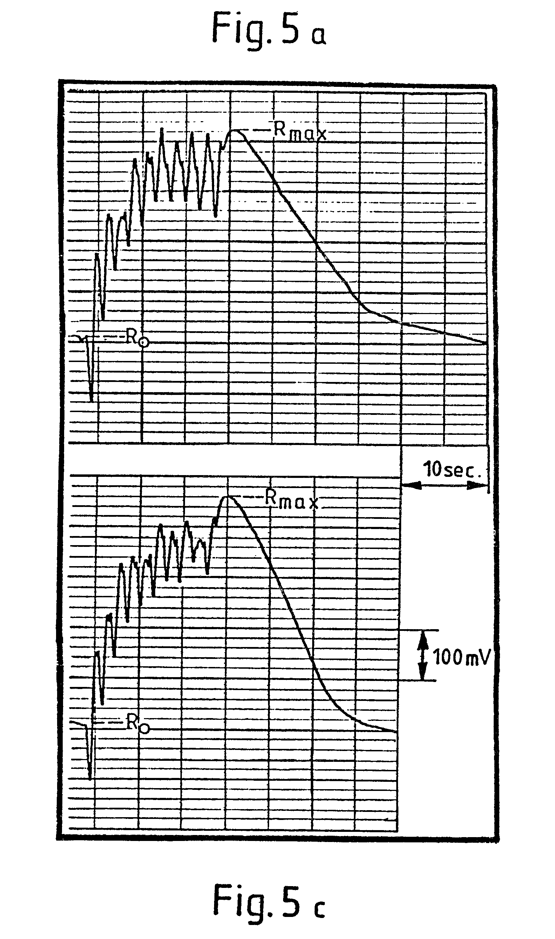

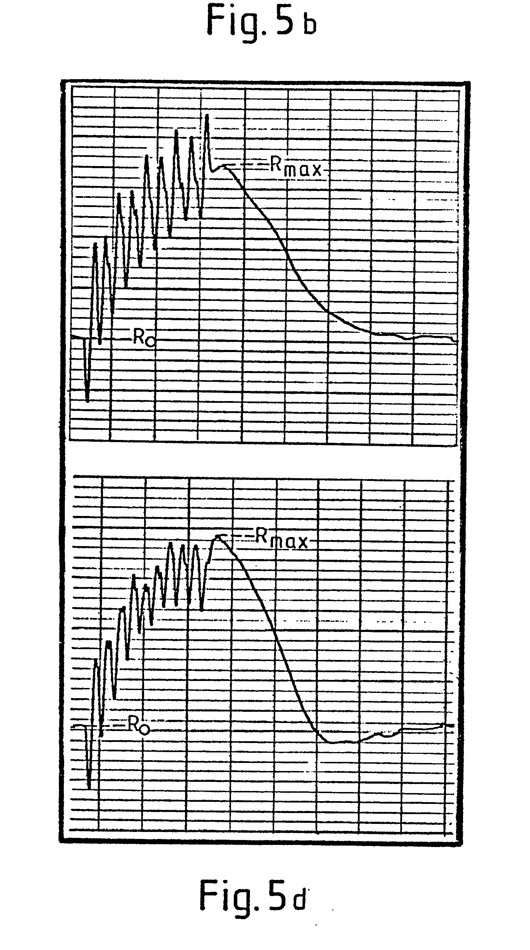

In der Figur 1 ist ein Ausführungsbeispiel eines erfindungsgemässen Messkopfes dargestellt. Figur 2 zeigt ein Blockschaltbild der Sende- und Auswerteelektronik. In den Figuren 3, 4 und 5 sind schliesslich einige ausgewählte Ergebnisse dargestellt.

Der Messkopf 1 muss kleine Abmessungen aufweisen, geringes Gewicht haben und muss auf der zu untersuchenden Extremität einfach fixiert werden können. Dabei darf kein Druck auf die Haut ausgeübt werden, da sonst das Blut aus den Hautgefässen weggedrückt wird und damit ein verfälschtes Messergebnis registriert würde. Der Messkopf darf den Patienten nicht bei den – nach Anlegen des Messkopfes – durchzuführenden Bewegungen behindern.

Die Licht-Reflexions-Rheographie soll in jeder Körperlage durchfürbar sein. Diese Forderung ist z.B. bei dem Verfahren der Plethysmographie nicht zu erfüllen.

In dem in Figur 1 dargestellten Ausführungsbeispiel ist der LRR-Messkopf aus einem PVC-Material gefertigt. Der Durchmesser des Messkopfes beträgt etwa 30 mm und sein Gesamtgewicht inkl. der Bauelemente 8, 9 und 10 nur etwa zehn Gramm.

Zur Befestigung an dem ausgewählten Hautareal können z.B. handelsübliche, beiderseits klebende Folienringe, Art. Nr. 2373579 E 300 der Firma Siemens verwendet werden, die üblicherweise zur Befestigung von EKG-Elektroden eingesetzt werden. Diese Folienringe werden zuerst auf die Stirnfläche 4 des Messkopfes 1 aufgelegt; anschliessend wird der Messkopf 1 auf die Haut fixiert.

Der Messkopf weist mehrere Öffnungen auf. Durch die Öffnung 3 ist ein mehradriges elektrisches Kabel zur Verbindung des Messkopfes 1 mit der Auswerteelektronik geführt. Die Rückseite des Messkopfes 1 ist mit einem Deckel 2 abgedeckt.

In der Achsenmitte des Messkopfes 1 ist in der Öffnung 6 ein Strahlungsempfänger 9 (Halbleiterphotodiode oder Phototransistor) befestigt.

Ringförmig um den Strahlungsempfänger 9 sind vorzugsweise drei (minimal 2) oder mehr Strahlungsquellen 8 (Halbleiterlaserdioden oder lichtemittierende Dioden, sog. LED's) in den Öffnungen 5 angebracht. Die Strahlungsquellen sind selektive Strahler, sie emittieren je nach Typ im nahen IR-Bereich oder im sichtbaren Bereich des Spektrums. Sie werden moduliert, um eine Beeinflussung der Messergebnisse durch Fremdlicht auszuschliessen. Die Strahlungsquellen dürfen keine Wärme entwickeln, weil dies die Messgenauigkeit beeinflussen würde.

Sowohl die Strahlungsquellen 8 als auch der Strahlungsempfänger 9 sind vorzugsweise mit Linsen ausgestattet.

Durch diese konstruktive Massnahme besitzen diese Bauelemente einen kleinen Strahlungsöffnungswinkel, so dass die emittierte selektive optische Strahlung einerseits tief in die Haut eindringen kann, andererseits kann der Strahlungsempfänger die von dem Hautgefässplexus reflektierten bzw. zurückgestreuten Strahlungsanteile (und nicht die Hautoberflächenreflexion) erfassen.

Durch die Verwendung mehrerer, ringförmig um den Detektor 9 befestigter Strahlungsquellen 8 wird, im Gegensatz zu den bekannten photoelektrischen plethysmographischen Aufnehmern, eine homogene Hautdurchleuchtung ohne Temperaturbelastung der Haut bewirkt.

Schliesslich befindet sich im Messkopf 1 ein kleiner Temperaturfühler 10 zur Messung der aktuellen Hauttemperatur. Er ist in der Öffnung 7 befestigt. Durch diese, in diesem Zusammenhang neue Massnahme wird erstmalig bei der angiologischen Messtechnik der an sich seit langem bekannten Beobachtung (vergl. z.B. Klin. Wschr. 34 [1956], 356) Rechnung getragen, dass der diagnostizierte Grad von venösen Abflussstörungen auch wesentlich von der Hauttemperatur abhängt.

Ein Beispiel der optoelektronischen Auswerteelektronik nach der Erfindung ist in Fig. 2 dargestellt. In dem Blockschaltbild nach Fig. 2 bedeuten:

11 ... Sinusgenerator ($f_o$ = 10 kHz beispielsweise)
12 ... Hochpass zur Unterdrückung tieffrequenter Störungen
13 ... Modulator und Regler zur symetrischen Ansteuerung der Strahlungsquellen 8
14 ... Verstärker des vom Strahlungsempfänger 9 detektierten Messsignals
15 ... Bandpass (selektive Strahlungsdetektion)
16 ... Demodulator
17 ... Tiefpass mit einstellbarer Grenzfrequenz zur Unterdrückung störender Einflüsse (wie z.B. Bewegungsartefakte, Störmodulation des Messsignals durch Atem- bzw. Pulswelle)
18 ... Eicheinheit, Nullpunkteinstellung
19 ... Auswerteeinheit des Messsignals (z.B. Schreiber zur Registrierung der dynamischen Hautreflexionsänderung, rechnergestützte Auswertung und Digitalanzeige der weiter unten erklärten Parameter $R_o$, $R_{max}$, $t_o$ usw.)

20 ... Aufarbeitung und Verstärkung des vom Temperaturfühler 10 gelieferten Messsignals
21 ... Analog/Digital-Wandler
22 ... Digitale Anzeigeeinheit der Hauttemperatur
23 ... Taktgebereinheit für das Bewegungsprogramm
24 ... Optischer Signalgeber zur Unterstützung des Ablaufes des Bewegungsprogrammes
25 ... Akustischer Signalgeber zur Unterstützung des Ablaufes des Bewegungsprogrammes

Die erfindungsgemässe Messeinrichtung zur nichtinvasiven Licht-Reflexions-Rheographie (LRR) wird in der Analogie zu der invasiven Venendruckmessung wie folgt angewandt:

Dem vorzugsweise sitzenden Patienten mit hängenden Beinen wird der Messkopf auf die Innenseite des relaxierten Beines in einem definierten Abstand (z.B. 10 cm) vom Innenknöchel appliziert. Der Messkopf ist dabei durch die bereits beschriebenen, beiderseits klebenden Ringe in einer definierten, gut reproduzierbaren Weise befestigt. Durch die Eicheinheit 18 mit entsprechender Elektronik wird anschliessend die Auswerteelektronik automatisch geeicht. Analog zu dem Ruhedruck $P_o$ wird bei der LRR der Ruhe-Hautreflexionsgrad $R_o$ registriert. Aufgezeichnet wird ausserdem automatisch auch die Temperatur des Hauttestareals unter dem Messkopf 1.

Auf Knopfdruck wird die Registriereinheit (vorzugsweise ein Schreiber) in Gang gesetzt; die LRR-Kurve wird aufgezeichnet. Nun wird das Bewegungsprogramm vom Patienten durchgeführt. Dabei folgt der Patient den optischen und akustischen Signalen der Taktgebereinheit 23, 24, 25 und führt innerhalb von 15 sec 10 maximale Dorsalflexionen im Sprunggelenk aus. Auch andere Bewegungsprogramme sind möglich. Danach wird das Bein wiederum in relaxierter Ruhelage hängen gelassen. Infolge des Bewegungsprogrammes (Sprunggelenkspumpe) entleeren sich zunächst die Hautgefässe, was zum Ansteigen des Hautreflexionsgrades bzw. zum Aufhellen der Haut führt. Diese Hautreflexionsänderung wird von der Registriereinheit angezeigt. In der Analogie zum dem Druck $P_{min}$ wird der Hautreflexionsgrad $R_{max}$ registriert. Nach Beendigung des Bewegungsprogrammes füllen sich die Venen normalerweise wieder nur durch den arteriellen Einstrom auf, d.h., die Hautreflexion sinkt in die Nähe des Ruhereflexionsgrades $R_o$. Bei venös Insuffizienten kommt es durch zusätzlichen venösen Reflux zur schnelleren Auffüllung der Gefässe.

Die Auffüllzeit $t_o$ und die Differenz des Hautreflexionsgrades $\triangle R = R_{max} - R_o$ können entweder graphisch aus der LRR-Kurve (s. Fig. 3–5) abgelesen werden oder sie können, wie auch andere mögliche Bewertungsfaktoren (z.B. die Steilheit der Hautreflexionsabnahme nach Beendigung des Bewegungsprogrammes), elektronisch mit Hilfe der rechnerunterstützten Auswerteeinheit ermittelt werden.

In der Fig. 3 ist die LRR-Kurve eines gesunden weiblichen Probanden aufgeführt. Gemessen wurde am rechten Unterschenkel, die Hauttemperatur betrug 32°C. Die Differenz des Hautreflexionsgrades $\triangle R$ entspricht hier der Spannungsdifferenz von 220 mV; die Auffüllzeit beträgt = 80 sec.

In der Fig. 4 sind die LRR-Kurven eines männlichen Patienten dargestellt; linkes Bein (links) gesund, T = 32,8°C; rechtes Bein (rechts) mit primärer Varikose, T = 33°C. Am linken Bein beträgt $\triangle R \triangleq 240$ mV; $t_o \sim 45$ sec; am rechten Bein ist dagegen $\triangle R \triangleq 150$ mV; $t_o \sim 20$ sec. Am erkrankten Bein ist insbesondere die kürzere Auffüllzeit $t_o$ pathognomonisch für die erklärte Diagnose.

In der Fig. 5 sind schliesslich Ergebnisse der Untersuchung bei einem Patienten mit CVI I. Grades dargestellt. Die Hauttemperatur ist in diesem Beispiel als Parameter aufgeführt:

Fig. 5
a: $T_{Haut}$ = 31°C (gekühlt) $\triangle R \triangleq 410$ mV; $t_o$ = 30 sec
b:     = 33°C (normal)     $\triangleq 340$ mV;     = 25 sec
c:     = 35°C (erwärmt)     $\triangleq 450$ mV;     = 19 sec
d:     = 39°C (erwärmt)     $\triangleq 380$ mV;     = 11 sec.

Experimentell wurde eine sehr gute Übereinstimmung zwischen den Ergebnissen der LRR-Messeinrichtung und den Ergebnissen der konventionellen, blutigen Venendruckmessung festgestellt, wobei die Nachteile der bisher bekannten und angewendeten Einrichtungen eliminiert werden konnten.

Bei der Anwendung der erfindungsgemässen Messeinrichtung zur Feststellung arterieller Durchblutungsstörungen werden nach der Befestigung des Messkopfes an dem zu untersuchenden Körperteil die beschriebenen Messungen unter gleichzeitiger Durchführung der sog. Lagerungstests (z.B. Kipptest, Allen-Test, Lagerungsprobe nach Ratschow) durchgeführt. Mittels der LRR-Einrichtung kann die aktuelle Hautdurchblutung im Bereich des Messareals viel exakter als durch die bisher übliche visuelle Beobachtung bestimmt und daraus auf etwaige Durchblutungsstörungen geschlossen werden.

Bei anderen Untersuchungen, z.B. im Zusammenhang mit arteriellen Durchblutungsstörungen im Bereich der unteren Extremitäten, kann es zweckmässig sein, an die elektronische Auswerteschaltung der Messeinrichtung gleichzeitig zwei oder mehrere Messköpfe anzuschliessen. Dadurch wird es möglich, die Differenz der Messwerte zwischen normal durchbluteten und unterversorgten Hautpartien zu ermitteln.

**Patentansprüche**

1. Messeinrichtung zur nichtinvasiven Feststellung peripherer Abfluss- und Durchflussstörungen in menschlichen Extremitäten, wobei die Blutentleerung bzw. -auffüllung der Venen oder Arterien eines Extremitätenareals in ihrem zeitlichen Verlauf erfassbar ist, mit einem leichten, an einem zu untersuchenden Hautareal mit doppelt klebenden Folienringen zu befestigenden Messkopf (1), der Öffnungen aufweist, in denen ein optischer Strahlungsempfänger (9) und eine Strahlungsquelle angeordnet sind, wobei der Strahlungsempfänger (9) und die Strahlungsquelle unmittelbar die Hautoberfläche berühren, und mit einer Auswerteschaltung für das Ausgangssignal des Strahlungsempfängers, dadurch gekennzeichnet, dass die Strahlungsquelle aus mehreren ringförmig um den Strahlungsempfänger angeordneten Strahlungssendern (8) besteht, die ein quasi homogenes optisches Strahlungsfeld tief in der Haut unter dem Messkopf (1) erzeugen, dass die Strahlungssender (8) selektive Strahler sind, die mit dem gleichen Signal moduliert sind, und die Strahlung gleicher Wellenlänge emittieren, die die Haut thermisch nicht belastet, und deren Wellenlänge im nahen IR-Bereich oder im sichtbaren Bereich des Spektrums liegt, und dass die elektronische Auswerteschaltung, neben Schaltkreisen zum selektiven Erfassen und Aufzeichnen des zeitlichen Verlaufs des reflektierten bzw. zurückgestreuten Strahlungsanteils bei gleichzeitiger Unterdrückung des Fremdlichts und der arteriellen Pulsation (Herzfrequenz) auch Taktgebereinheiten (23, 24, 25) zur optischen und akustischen Führung des Patienten bei der Durchführung eines vorgegebenen Bewegungsprogrammes enthält.

2. Messeinrichtung nach Anspruch 1, dadurch gekennzeichnet, dass der Messkopf (1) eine weitere Öffnung (7) aufweist, in der ein kleiner Temperaturfühler (10) zur gleichzeitigen Messung der Hauttemperatur im abgedeckten Messareal angeordnet ist.

3. Messeinrichtung nach Anspruch 1 oder 2, dadurch gekennzeichnet, dass die Strahlungssender (8) und der Strahlungsempfänger (9) mit integrierter Fokussieroptik ausgestattet sind.

4. Messeinrichtung nach einem der Ansprüche 1 bis 3, gekennzeichnet dadurch, dass der Messkopf (1) in einem flachen, kreisrunden Gehäuse angeordnet ist, das einen Aussendurchmesser von vorzugsweise etwa 30 mm aufweist.

5. Messeinrichtung nach einem der Ansprüche 1 bis 4, gekennzeichnet durch eine solche Ausbildung der elektronischen Auswerteschaltung, dass aus den Signalen eines oder mehrerer gleichartiger Messköpfe die Auffüllzeit $t_o$, die Differenz des Hautreflexionsgrades $\triangle R = R_{max} - R_o$ und/oder die Steilheit der Hautreflexionsabnahme entweder graphisch oder analytisch gewonnen werden können.

6. Messeinrichtung nach einem der Ansprüche 2 bis 5, dadurch gekennzeichnet, dass die Auswerteelektronik Baugruppen (20, 21, 22) zur Auswertung des vom Messkopf (1) gelieferten Temperatursignals sowie zur digitalen Anzeige der jeweiligen Temperatur des Hautareals aufweist.

## Claims

1. Non-invasive measuring device for locating drainage and circulation malfunctions in veins and arteries, the emptying or filling of blood in the veins or arteries of an area on the respective extremity being detectable in its progress as a function of time, with a light measuring head (1) which can be affixed to an area of the skin to be examined by means of double-adhesive foil rings and which has openings in which an optical radiation receiver (9) and a source of radiation are disposed, the radiation receiver (9) and the source of radiation directly contacting the surface of the skin, and with an evaluation circuit for the output signal of the radiation receiver, characterised in that the source of radiation consists of a plurality of radiation transmitters (8) arranged annularly about the radiation receiver and producing a quasi homogeneous optical radiation field deep in the skin below the measuring head (1), that the radiation transmitters (8) are selective radiators which are modulated with the same signal and emit radiation of the same wave length which does not subject the skin to thermal strain and the wave length thereof lies in the near infrared or in the visible range of the spectrum and that the electronic evaluation circuit in addition to circuits for selectively detecting and recording the progress of the reflected or dispersed back amount of radiation as a function of time whit a simultaneous suppression of the outside light and the arterial pulsation (heart frequency) also includes clocking units (23, 24, 25) for optically or audibly assisting the patient in performing a predetermined movement programme.

2. Measuring device as claimed in claim 1, characterised in that the measuring head (1) has a further opening (7) in which a small temperature sensor (10) is disposed for simultaneously measuring the temperature of the skin in the covered measuring area.

3. Measuring device as claimed in claim 1 or 2, characterised in that the radiation transmitters (8) and the radiation receiver (9) are equipped with an integrated focusing optical system.

4. Measuring device as claimed in any one of claims 1 to 3, characterised in that the measuring head (1) is disposed in a flat, circular housing which has an external diameter of preferably approximately 30 mm.

5. Measuring device as claimed in any one of claims 1 to 4, characterised by such a construction of the electronic evaluation circuit that from the signals of one or several similar measuring heads the filling time $t_o$, the difference of the degree of skin reflection $\triangle R = R_{max} - R_o$ and/or the slope of the skin reflection decrease can be obtained either graphically or analytically.

6. Measuring device as claimed in any one of claims 2 to 5, characterised in that the evelation circuit includes assemblies (20, 21, 22) for evaluating the temperature signal supplied by the measuring head (1) and for digitally displaying the respective temperature of the area of the skin.

## Revendications

1. Appareil de mesure destiné à la détermination limitée ou locale de perturbations de l'écoulement sanguin et de l'irrigation sanguine au niveau périphérique dans des extrémités du corps humain, l'évacuation du sang veineux et l'arrivée du sang artériel d'une région d'extrémité pouvant respectivement être relevées dans leur déroulement dans le temps, l'appareil de mesure comportant une tête de mesure légère (1), à fixer à une région de la peau à soumettre à l'examen, à l'aide d'anneaux en feuille de métal à adhérence double, tête de mesure (1) qui présente des ouvertures dans lesquelles sont montés un récepteur de rayonnement optique (9) et une source de rayonnement, le récepteur de rayonnement (9) et la source de rayonnement étant en contact direct avec la surface de la peau, et l'appareil comportant en outre un montage d'évaluation ou appréciation pour le signal de sortie du récepteur de rayonnement, l'appareil de mesure étant caractérisé en ce que la source de rayonnement est formée de plusieurs émetteurs de rayonnement (8) disposés en anneau autour du récepteur de rayonnement, émetteurs de rayonnement qui produisent, profondément dans la peau, en dessous de la tête de mesure (1), un champ de rayonnement optique quasi homogène, en ce que les émetteurs de rayonnement (8) sont des émetteurs de rayonnement sélectifs qui sont modulés par le même signal et émettent un rayonnement de même longueur d'onde qui ne soumet la peau à aucun effet thermique et dont la longueur d'onde se situe dans la zone voisine du rayonnement IR ou dans la zone visible du spectre, et en ce que le montage électronique d'évaluation ou appréciation comporte, outre des circuits de commande permettant de relever de façon sélective et d'enregistrer l'allure dans le temps de la partie du rayonnement réfléchie ou diffusée en retour, sous suppression simultanée de la lumière étrangère, et la pulsation du sang artériel (fréquence cardiaque), des unités émettrices de rythme (23, 24, 25) servant à guider le patient de façon optique et acoustique lorsqu'il exécute un programme de mouvements donné.

2. Appareil de mesure suivant la revendication 1, caractérisé en ce que la tête de mesure (1) présente une autre ouverture (7) dans laquelle est montée une petite sonde de température (10) permettant la mesure simultanée de la température de la peau dans la zone de mesure couverte.

3. Appareil de mesure suivant l'une ou l'autre des revendications 1 et 2, caractérisé en ce que les émetteurs de rayonnement (8) et le récepteur de rayonnement (9) sont munis d'une lentille de focalisation integrée.

4. Appareil de mesure suivant l'une quelconque des revendications 1 à 3, caractérisé en ce qu'il (1) est monté dans un boîtier plat, de forme circulaire, qui présente un diamètre externe avantageusement d'environ 30 mm.

5. Appareil de mesure suivant l'une quelconque des revendications 1 à 4, caractérisé en ce que le montage électronique d'évaluation ou appréciation est prévu sous une forme telle qu'à partir des signaux d'une ou de plusieurs têtes de mesure du même genre, la durée de l'arrivée du sang $t_o$, la différence du degré de réflexion de la peau $\triangle R = R_{max} - R_o$ et/ou l'allure de la diminution de la réflexion de la peau peuvent être obtenues par moyen graphique ou analytique.

6. Appareil de mesure suivant l'une quelconque des revendications 2 à 5, caractérisé en ce que le montage électronique d'évaluation ou appréciation comporte des groupes fonctionnels (20, 21, 22) servant à l'évaluation ou appréciation du signal de température fourni par la tête de mesure (1), de même qu'à l'indication digitale de la température particulière de la région de la peau.

Fig.1

Fig. 2

Fig.3

50mV

$R_{max}$

$R_0$

10sec.

0 063 649

Fig. 4

100mV

10sec.

Rmax

Ro

15

# Fig. 5 a

# Fig. 5 c

# Fig. 5 b

# Fig. 5 d